# EUROPEAN PATENT APPLICATION

(11) **EP 4 458 421 A1**
(43) Date of publication of application: **06.11.2024**
(21) Application number: 22915972.8
(22) Date of filing: 23.12.2022
(51) Int. Cl.: A61Q 1/00, A61Q 1/10, A61K 8/06, A61K 8/26, A61K 8/73, A61K 8/81

(54) **AQUEOUS LIQUID COSMETIC MATERIAL**

(30) Priority: 28.12.2021 JP 2021214016
(71) Applicant: MITSUBISHI PENCIL COMPANY, LIMITED, Tokyo 140-8537 (JP)
(72) Inventor: SHINOHARA Ryuichi, Fujioka-shi, Gunma 375-8501 (JP); YAMAZAKI Yuichi, Fujioka-shi, Gunma 375-8501 (JP)
(74) Representative: Vossius & Partner Patentanwälte Rechtsanwälte mbB
(86) International application number: PCT/JP2022/047719
(87) International publication number: WO 2023/127749

(57) **Abstract**

The present disclosure provides an aqueous liquid cosmetic material suitable for makeup cosmetic materials for eyeshadows, eyeliners, eyebrow makeup, mascaras, and the like that, even when containing a larger amount of a flaky pigment such as a glittering pigment than the known ones and made into a low-viscosity aqueous blending composition, can still greatly improve the intensity of lines drawn using the cosmetic material without causing aggregation or settling of the flaky pigment while also achieving an improved adhering property. The aqueous liquid cosmetic material according to the present disclosure is a cosmetic material accommodated in a pen-type applicator A having a brush 23 at an application portion, the aqueous liquid cosmetic material containing at least a flaky pigment A, 1 to 9 mass% of a flaky pigment B that is surface-treated with at least one type of crystalline cellulose, a dextrin, a thickening polysaccharide, emulsion particles containing 4 to 10 mass% of an acrylate copolymer in terms of solid content, and water.

## Description

### Technical Field

The present specification related to an aqueous liquid cosmetic material that, even when containing a larger amount of a flaky glittering pigment than the known ones and made into a low-viscosity aqueous blending composition, can still greatly improve the intensity of the lines drawn using the cosmetic material and can also achieve an improved adhering property without causing aggregation or settling of the flaky pigment.

### Background Art

In the related art, when glittering particles having a large particle size are blend into a cosmetic material having fluidity, there are the usual ways of giving the cosmetic material itself a very high viscosity, or making the cosmetic material into a gel in order to prevent aggregation or settling of particles. For example, in Patent Document 1, in a cosmetic material containing water, prevention of aggregation or settling of particles is addressed by preparing a gel cosmetic material containing the following components (a) to (c): (a) 0.1 to 3 mass% of pectin; (b) 0.0025 to 0.75 mass% of a salt that generates polyvalent cations in an aqueous solution; (c) 0.01 to 3 mass% of polyvinylpyrrolidone and/or a copolymer of vinylpyrrolidone and another polymerizable monomer; (d) water-soluble polyoxyethylene polyoxypropylene glycol; and (e) glittering particles. By making the cosmetic material into a gel or giving the cosmetic material a very high viscosity, the settling rate of particles slows down, and the particles are constantly surrounded by solvent, thereby preventing aggregation or settling.

Meanwhile, a prior application filed by the present applicants discloses, as an aqueous nail polish composition, a low-viscosity composition containing a flaky pigment whose surface is coated with a water-insoluble substance such as crystalline cellulose (see, for example, Patent Document 2) . Patent Document 2 describes that in the aqueous nail polish composition, redispersibility is improved to some extent not because aggregation or settling of a flaky pigment is suppressed but because the surfaces are coated with crystalline cellulose or the like, and that the aqueous nail polish composition aims to improve the outflow from a so-called plastic feed, that is, a tubular feed. In the aqueous nail polish composition of Patent Document 2, because of the coating with crystalline cellulose, there was no problem in redispersibility after approximately one month in an applicator having a stirring ball inside an application liquid storage portion. In terms of changes over a long period of time, only slight aggregation or settling was observed. In the applicator having an application portion that is a brush, slight differences in the hue between an upper part and a lower part within the brush can be seen sporadically over a long period of time.

Furthermore, the present applicants has disclosed a liquid cosmetic material composition having a low viscosity composition. In order to obtain a higher glittering property, the present applicants added a specific clay mineral and an acrylic copolymer in the liquid cosmetic material composition in addition to a flaky pigment whose surface is coated with a water-insoluble substance such as crystalline cellulose. The liquid cosmetic material composition is stored in an applicator having an application portion that is a brush, and when a user uses the brush to apply the liquid cosmetic material composition, the applied shear force causes the flaky pigment to slide even if they overlap, resulting in a widely spread coating film (see, for example, Patent Document 3). Patent Document 3 describes an effect of a high glittering property being exhibited "with just a small addition amount" of a flaky pigment (paragraph [0038]). That is, redispersion is facilitated by the reduced addition amount of the flaky pigment, while the glittering property after application can be seen as unchanged compared to that in the aforementioned Patent Document 2 or the like. Patent Document 3 also discloses a formulation containing a flaky pigment whose surface is coated with a water-insoluble substance such as crystalline cellulose, a thickener such as xanthan gum, and a dextrin (Example 3). However, the addition amount is very small, and the technical idea is different from that for overcoming the problem of aggregation or settling due to addition of a large amount of the flaky pigment.

Meanwhile, maltodextrin and/or dextrose has been used as a gloss enhancer containing a flaky glittering pigment (see, for example, Patent Document 4) . Patent Document 4 is a component of a dry powder mixture for coating a product surface, and is not for enhancing luster of the flaky glittering pigment themselves.

In addition, a prior application filed by the present applicants discloses an ink composition containing a flaky glittering pigment accommodated in a slim pen barrel, but does not contain disclosure of a liquid cosmetic material. Only a small and light-weight stirrer can be enclosed in the slim pen barrel, and therefore redispersion of the pigment in this ink composition becomes very difficult once solid contents such as pigments or the like form a sediment called "hard cake". To obtain an ink composition having excellent redispersibility even with a small and light-weight stirrer, a solution is to add crystalline cellulose and a flaky pigment having a different average particle size in the same system so that the sediment has voids, (see, for example, Patent Document 5) . The formulation of Patent Document 5 contains a large amount of water-soluble resin. As such, although the viscosity of the ink composition is relatively close to Newtonian, the viscosity still inevitably becomes slightly high (exceeding 100 mPa·s), and the technical idea is basically different from the blending composition and the like of an aqueous liquid cosmetic material.

### Citation List

### Patent Document

Patent Document 1: JP 2004-300053 A (Claims and the like)
Patent Document 2: JP 2013-124227 A (Claims and the like)
Patent Document 3: JP 2021-14412 A (Claims and the like)
Patent Document 4: JP 2009-536231 T (Claims, Paragraph 0082, and the like)
Patent Document 5: JP 2011-84661 A (Claims and the like)

### Summary of Invention

### Technical Problem

The present disclosure has been made as a result of intensive research in view of the problems and current situation of the prior art described above. An object of the present disclosure is to provide an aqueous liquid cosmetic material suitable for makeup cosmetic materials for eyeshadows, eyeliners, eyebrow makeup, mascaras, and the like that, surprisingly, even when containing a larger amount of a flaky glittering pigment than the known ones and having a low-viscosity aqueous composition, can still greatly improve the intensity of lines drawn using the cosmetic material, and achieve an improved adhering property without causing aggregation or settling of the flaky glittering pigment.

### Solution to Problem

As a result of intensive studies in view of the above known problems and the like, the present inventors have found an aqueous liquid cosmetic material accommodated in a pen-type applicator having a brush at an application portion. The aqueous liquid cosmetic material having a blending composition containing at least a flaky pigment, a specific amount of a surface-treated flaky pigment, a dextrin, a thickening polysaccharide, a specific amount of emulsion particles containing an acrylate copolymer, and water, thus completing the present disclosure.

That is, the aqueous liquid cosmetic material according to the present disclosure is a cosmetic material accommodated in a pen-type applicator having a brush at an application portion, the aqueous liquid cosmetic material containing at least a flaky pigment A, 1 to 9 mass% of a flaky pigment B that is surface-treated with at least one type of crystalline cellulose, a dextrin, a thickening polysaccharide, emulsion particles containing 4 to 10 mass% of an acrylate copolymer in terms of solid content, and water.

The dextrin is preferably at least one selected from Group X below. Group X: powdered syrup, maltodextrin, dextrin, and cyclic dextrin The thickening polysaccharide is preferably at least one selected from Group Y below.

Group Y: xanthan gum, carrageenan, guar gum, locust bean gum, tamarind gum, cationic guar gum, and quince seed

The flaky pigment A is preferably a silica-coated pigment. The flaky pigment A preferably contains an untreated flaky pigment having a different particle size. Further, the flaky pigment B preferably contains a flaky pigment having a different particle size and surface-treated with crystalline cellulose.

Further, the particle size of the flaky pigment A and the particle size of the flaky pigment B are each 1 to 50 µm or 51 to 100 µm, and the difference therebetween is 30 µm or greater.

In the present specification, the term "aqueous" refers to a composition of a uniform system (not a W/O emulsion, an O/W emulsion, etc.) in which water and/or a water-soluble organic solvent is the main solvent. Advantageous Effects of Invention

The present disclosure provides an aqueous liquid cosmetic material suitable for makeup cosmetic materials for eyeshadows, eyeliners, eyebrow makeup, mascaras, and the like that, even when containing a larger amount of a flaky pigment such as a glittering pigment than the known ones and made into a low-viscosity aqueous blending composition, can still greatly improve the intensity of lines drawn using the cosmetic material and achieve an improved adhering property without causing aggregation or settling of the flaky pigment.

The object and effects of the present disclosure can be recognized and obtained especially using the components and combinations indicated in the claims. Both general explanation described above and detailed explanation described below are exemplary and explanatory and do not limit the present disclosure described in Claims.

### Brief Description of Drawings

FIG. 1 is a vertical cross-sectional view illustrating an example of a liquid cosmetic material applicator accommodating an aqueous liquid cosmetic material of the present disclosure and illustrating a state before use.
FIG. 2 is a vertical cross-sectional view illustrating the liquid cosmetic material applicator of FIG. 1 in a state at the beginning of use.
FIG. 3 is a partial vertical cross-sectional view illustrating another example of the liquid cosmetic material applicator accommodating the aqueous liquid cosmetic material of the present disclosure.
FIG. 4 is a partial vertical cross-sectional view illustrating another example of the liquid cosmetic material applicator accommodating the aqueous liquid cosmetic material of the present disclosure.

### Description of Embodiments

Embodiments of the present disclosure will be described below in detail. However, note that the technical scope of the present disclosure is not limited to the embodiments described below and includes the invention described in Claims and equivalents thereof. In addition, the present disclosure can be implemented based on the contents disclosed in the present specification and technical common knowledge (including design matters and obvious matters) in the art.

The aqueous liquid cosmetic material according to the present disclosure is a cosmetic material accommodated in a pen-type applicator having a brush at an application portion, the aqueous liquid cosmetic material containing at least a flaky pigment A, 1 to 9 mass% of a flaky pigment B that is surface-treated with at least one type of crystalline cellulose, a dextrin, a thickening polysaccharide, 4 to 10 mass% in terms of solid content of emulsion particles containing an acrylate copolymer, and water.

The flaky pigment used in the present disclosure incluses at least two types of flaky pigments, the flaky pigment A and the flaky pigment B which is surface-treated with at least one type of crystalline cellulose. Examples of the flaky pigment A to be used include flaky pigments such as an aluminum flake pigment (aluminum powder pigment), iron oxide-coated mica titanium, mica titanium, blue-coated mica titanium, glass flakes coated with metal or metal oxide, pearl pigment, and aluminum-coated polyester film.

Specific examples of the flaky pigment A to be used include flaky pigments such as commercially available aluminum flake pigment, metal oxide-coated glass flakes (trade name "METASHINE" and the like), and metal oxide-coated mica (trade name "Lumina" and the like).

The flaky pigment B surface-treated with at least one type of crystalline cellulose is obtained by subjecting a surface-untreated flaky pigment such as the flaky aluminum pigment A, iron oxide-coated mica titanium, mica titanium, blue-coated mica titanium, or metal or metal oxide-coated glass flakes, to a surface treatment with at least one type of crystalline cellulose.

Crystalline cellulose is a cellulose crystallite aggregate having a substantially certain degree of polymerization obtained by acid hydrolysis or alkali oxidative decomposition of cellulose. Unlike known aqueous solutions of thickening polysaccharides or the like, crystalline cellulose has a property that cellulose crystals become dispersed particles in water. The flaky pigment B of the present disclosure is a pigment in which the above-described surface-untreated flaky pigment is surface-treated with at least one type of crystalline cellulose having the above-described property.

Examples of the crystalline cellulose used for the surface treatment include a colloidal grade obtained by subjecting surfaces of fine cellulose crystals of primary particles to a coating treatment with a water-soluble polymer and the like. The specific examples thereof include Ceolus RC-591, RC-N81, RC-591NF, CL-611 and Ceolus Cream (all available from Asahi Kasei Corporation).

Examples of the method for coating the surface of the flaky pigment with crystalline cellulose include 1) a method of adhering crystalline cellulose to the surface of the flaky pigment by spray-drying a water slurry mixture of crystalline cellulose and the flaky pigment using a spray dryer (hereinafter referred to as "Production Method 1"), and 2) a method of dissolving the substance soluble in an acidic or basic aqueous solution while adjusting the pH, and precipitating crystalline cellulose on the surface of the flaky pigment by shifting the pH of a solution with an acid or baseto insolubilization (hereinafter referred to as "Production Method 2") .

When the surface coating amount of crystalline cellulose is too small, the effects of the present disclosure cannot be exhibited. Meanwhile, when the surface coating amount of crystalline cellulose is too large, the glittering property decreases. Therefore, it is desirable that the surface coating amount of crystalline cellulose is in the range of 0.1 to 10%, preferably 0.5 to 8%, and more preferably 1 to 7%, as a surface coating ratio of the flaky pigment (calculated from mass% at the time of surface treatment).

In the present disclosure, the flaky pigment A may be the aluminum flake pigment, iron oxide-coated mica titanium, mica titanium, blue-coated mica titanium, and the like, which are surface-untreated and may also be a silica-coated flaky pigment whose surface is treated with silica (SiO2). Preferably, these flaky pigments A are untreated flaky pigments having different average particle sizes and/or silica-coated flaky pigments. The silica-coated flaky pigment may be silica particles or the like subjected to the above-described Production Methods 1 or 2 (including the range of the above-described surface coating ratio and the like) or commercially available products if available. Examples of commercially available products include the EMR series (available from Toyo Aluminium K.K.) and SW-120PM (available from Asahi Kasei Chemicals Corporation) in which surfaces of aluminum flakes are coated with high-density silica. The flaky pigment B to be used are surface-treated with crystalline cellulose as described above, but since it is surface-treated with at least one type of crystalline cellulose, it may be surface-treated together with a dextrin and the like described later by the Production Methods 1 or 2. Further, the flaky pigment B may include two or more types of flaky pigments having an average particle size after surface-treated with at least one type of crystalline cellulose.

The average particle size of the flaky pigment A and the average particle size of flaky pigment B to be used can each be in the range of 1 to 100 µm. Preferably, the average particle size of the flaky pigment A and the average particle size of flaky pigment B are each 1 to 50 µm or 51 to 100 µm, and the difference therebetween is preferably 30 µm or greater. More preferably, the flaky pigment A has an average particle size in the range of 1 to 50 µm, and the flaky pigment B has an average particle size in the range of 51 to 100 µm, and the difference therebetween is 30 µm or greater.

Here, "the difference therebetween is 30 µm or greater" means that when the average particle size of the flaky pigment A is 10 µm, the average particle size of the flaky pigment B is 40 µm or greater, and when the average particle size of the flaky pigment A is 25 µm, the average particle size of the flaky pigment B is 55 µm or greater.

In the present disclosure (including Examples), the "average particle size" refers to a value measured and calculated by a dynamic light scattering method [particle size analyzer FPAR-1000, available from Otsuka Electronics Co., Ltd.]. Further, when the flaky pigment coated with crystalline cellulose or silica used in the present disclosure is observed with an electron microscope (× 35000 magnification), crystalline substances such as the above-mentioned crystalline cellulose can be confirmed on the surfaces.

In the present disclosure, while the flaky pigment A (untreated flaky pigment, silica coating-treated flaky pigment, and these flaky pigments having different average particle sizes) and the flaky pigment B (flaky pigment treated with at least one type of crystalline cellulose are used, and flaky pigments having different average particle sizes and treated with the crystalline cellulose), the content of the flaky pigment B is 1 to 9 mass% (hereinafter, "mass%" is simply referred to as "%") with respect to the total amount of the composition from the viewpoint of allowing the effect of the present disclosure to exhibit.

Preferably, the total content of the flaky pigment A and the flaky pigment B is greater than 1.0 to 10 mass% (hereinafter, "mass%" is simply referred to as "%"), more preferably greater than 1.0 to 8.0%, and particularly preferably 1.1 to 8.0% with respect to the total amount of the composition from the viewpoint of a sufficient glittering property and stable liquid discharge during product use.

The blending ratio of the flaky pigment A and the flaky pigment B is preferably 1:2 to 1:10, more preferably 1:3 to 1:5 on a mass ratio basis. When the total content is 1.0% or greater, a sufficient glittering property can be obtained, and when the total content is 10% or less, the liquid can be stably discharged.

The "dextrin" used in the present disclosure means a dextrin selected from the group consisting of acyclic dextrins in which α-glucose units are bonded in a linear or branched chain, cyclic dextrins in which α-glucose units are bonded in a cyclic form, and mixtures thereof. From the viewpoint of the settling property of the coloring material and stable liquid discharge, the dextrin is preferably at least one type selected from Group A below.

Group A: powdered syrup, maltodextrin, dextrin, cyclic dextrin Dextrins are classified as follows based on a numerical value indicating the degree of saccharification of starch, called DE (dextrose equivalent) . Powdered syrup (DE: about 20 to 40), maltodextrin (DE: about 10 to 20), and dextrin (DE: about 10 or less).

Examples of the cyclic dextrin include unsubstituted cyclic dextrins such as α-cyclodextrin, β-cyclodextrin and γ-cyclodextrin, and substituted cyclic dextrins such as hydroxypropylated β-cyclodextrin and maltosylcyclodextrin. Furthermore, any commercially available products of the dextrin of Group A can be used. Examples of commercially available products thereof include 103 Dextrin (available from Tokai Dextrin K.K.) and Celldex SL-20 (available from NIHON SHOKUHIN KAKO CO., LTD.).

The content of these dextrins is preferably 0.05 to 0.3% with respect to the total amount of the composition from the viewpoint of the settling property of the coloring material and stable liquid discharge.

When the content is less than 0.05%, the change in the degree of glittering of lines drawn after a certain length of time becomes large, and when the content exceeds 0.3%, the discharge amount of liquid decreases, which is not preferable.

The thickening polysaccharide used in the present disclosure can be any thickening polysaccharide without limitation. From the viewpoint of the settling property of the coloring material and stable liquid discharge, the thickening polysaccharide is preferably at least one type selected from Group Y below.

Group Y: xanthan gum, carrageenan, guar gum, locust bean gum, tamarind gum, cationic guar gum, quince seed

Moreover, any commercially available products of those of Group Y above can be used. Examples of the commercially available products include KELTRON CG (available from Sansho Co., Ltd.) and KELZAN T (available from Sansho Co., Ltd.).

The content of these thickening polysaccharides is preferably 0.01 to 0.1% with respect to the total amount of the composition from the viewpoint of the settling property of the coloring material, stable application, and the like.

When the content is less than 0.01%, the change in the degree of glittering of lines drawn after a certain length of time becomes large, and when the content exceeds 0.1%, the discharge amount of liquid decreases, which is not preferable.

In the present disclosure, the emulsion particles containing an acrylateacrylate copolymer are used in view of film formation. The emulsion particles containing an acrylateacrylate copolymer to be used may be a random copolymer, a graft copolymer, a block copolymer, or a core-shell copolymer. In particular, the emulsion particles are preferably an acrylic resin emulsion or a urethane-acrylic composite resin emulsion containing particles having a core-shell structure in which the core is an acrylic resin such as poly (meth) acrylate or a urethane resin such as polyurethane while the shell is an acrylic resin such as poly(meth)acrylate or a urethane resin such as polyurethane.

Specific examples include an alkyl acrylate copolymer or an acrylate copolymer which is (a dispersion of) emulsion particles containing an acrylate copolymer. Examples of an alkyl acrylate copolymer include a (octylacrylamide/hydroxypropyl acrylate/butylaminoethyl methacrylate) copolymer, alkyl acrylate copolymer ammonium (trade name "Yodosol GH800F": solid content 45%), and a styrene acrylate copolymer; styrene/alkyl acrylate copolymer emulsion (Yodosol GH41F (available from Akzo Nobel), Daitosol 5000STY (available from Daito Kasei Kogyo Co. Ltd.)), and core-shell type emulsion particles containing an acrylate copolymer; and emulsion of core-shell type copolymer of ethylhexyl acrylate/methacrylic acid copolymer [(acrylate/methylstyrene/styrene) copolymer ammonium] (EMUPOLY CE-119N (available from Gifu Shellac)).

In the emulsion particles containing these acrylate copolymers, usually, a resin component is finely dispersed in an aqueous component at a concentration of 20 to 60 mass% as a solid content. The blending amount thereof is 4 to 10 mass% in terms of solid content with respect to the total amount of the liquid cosmetic material.

When the content of the emulsion particles is less than 4 mass%, the adhesiveness and the water-resistant adhesiveness decrease, and meanwhile, when the content is greater than 10 mass%, the viscosity of the liquid cosmetic material increases and it becomes difficult to apply the liquid cosmetic material.

In the present disclosure, it is preferable to use an aqueous solvent, such as at least one of a lower alcohol having 6 or less carbons and a polyhydric alcohol (each alone or a mixture of two or more, the same applies hereinafter) in view of the drying property of lines drawn and resistance to microorganisms.

Specific examples of the lower alcohol having 6 or less carbons include at least one of methyl alcohol (methanol), ethyl alcohol (ethanol), n-propyl alcohol, isopropyl alcohol, n-butyl alcohol, sec-butyl alcohol, tert-butyl alcohol, isobutyl alcohol, pentyl alcohol, ethylene glycol, and propylene glycol.

Moreover, examples of the polyhydric alcohol include glycerin, erythritol, threitol, arabinitol, xylitol, ribitol, propylene glycol, 1,3-butylene glycol, 1,2-pentanediol, ethylene glycol, diethylene glycol, and pentaerythritol.

From the viewpoint of stability and resistance to microorganism, the total content of the aqueous solvent used is preferably 5 to 15%, more preferably 8 to 13%, with respect to the total amount of the liquid cosmetic material.

The water serving as the solvent used in the present disclosure can be distilled water, ion exchanged water, purified water, pure water, ultrapure water, or the like. From the viewpoint of adjusting stability over time and the drying property of lines drawn, the water is preferably 65% or greater, particularly preferably 68 to 75 mass%.

From the viewpoint of ensuring the solubility of alkali-soluble resins such as the alkyl acrylate copolymer and suppressing skin irritation, the aqueous liquid cosmetic material of the present disclosure preferably has a pH in the range of 6 to 9 when measured using a glass electrode. The pH can be adjusted by using a pH adjuster such as 2-amino-2-methyl-1-propanol, 2-amino-2-methyl-1,3-propanediol, triethanolamine, L-arginine, citric acid, aqueous ammonia, or sodium hydroxide, and particularly preferably citric acid or aminomethylpropanol (2-amino-2-methyl-1-propanol) .

Furthermore, the aqueous liquid cosmetic material of the present disclosure can contain, in addition to the above-mentioned components, optional components used in ordinary liquid cosmetic materials. Specifically, the aqueous liquid cosmetic material of the present disclosure may contain preservatives, antioxidants, ultraviolet absorbers, chelating agents, humectants, beauty ingredients, fragrances, the above dextrins, thickeners other than thickening polysaccharides, and other dispersants such as polyethylene glycol alkyl ethers in appropriate amounts within a range that does not impair the effects of the present disclosure. In addition to inorganic pigments such as carbon black and iron oxide pigments other than the flaky pigments described above, other coloring materials such as organic pigments, dyes such as Red 226, and resin particle pigments can be contained as necessary within a range that does not impair the effects of the present disclosure. In addition, polyoxyethylene behenyl (C22) ether (BB-20), polyoxyethylene (4 or less or greater than 20) cetyl (C18) ether (BC-2 or BC-23), or the like can be used from the viewpoint of stabilization. The thickener may contain, for example, a styrene acrylic copolymer as necessary.

The aqueous liquid cosmetic material of the present disclosure preferably has a viscosity of 15 mPa·s or less, preferably 10 mPa·s or less, more preferably 2 to 8 mPa·s.

When the viscosity exceeds 15 mPa·s, the outflow property of the aqueous liquid cosmetic material decreases significantly, which is not preferable.

The viscosity range can be adjusted by suitably combining various raw materials such as the flaky pigment to be used, the average particle size thereof, water-soluble acrylic copolymer, the emulsion particles containing an acrylate copolymer, and aqueous solvent, combining the contents in suitable ranges, or, using a suitable dispersion method. Note that, in the present disclosure, the viscosity measurement (including in Examples described below) was specifically carried out by measuring the viscosity of the obtained cosmetic composition at a predetermined shear rate (50 rpm) at a temperature of 25°C using a cone and plate viscometer (ELD viscometer among TV-30 viscometers, standard cone and plate, available from Tokimec).

A suitable aqueous liquid cosmetic material of the present disclosure can be prepared by adjusting at least the flaky pigment A, the flaky pigment B surface-treated with at least one type of crystalline cellulose, the dextrin, the thickening polysaccharide, the emulsion particles containing an acrylate copolymer, water, and other components under suitable dispersion conditions using a disperser such as a homomixer, a sand mill, an ultrasonic homogenizer, or a high-pressure homogenizer.

The obtained aqueous liquid cosmetic material can be made available for use by being accommodated (filled) in a pen-type liquid cosmetic material applicator (container) having a brush as an application portion.

The liquid cosmetic material applicator that can be used is not limited as long as it is a liquid cosmetic material applicator provided with a brush for eyeliner or eyebrow use, for example.

A preferred example is an applicator provided with an application body serving as an application means and consisting of an eyeliner or eyebrow brush (brush pen), and that has a container filled with a liquid cosmetic material.

The aqueous liquid cosmetic material of the present disclosure can be made available for use by being accommodated in a liquid cosmetic material applicator having an application portion provided with a brush. In the present disclosure, a suitable liquid cosmetic material applicator is not limited as long as it is a liquid cosmetic material applicator having an application portion provided with a brush and having a structure that allows a liquid cosmetic material composition (application liquid) to be suitably applied to the skin or applied as eyeliner, eyeshadow, eyebrow makeup suitable for focal point makeup to around the eyes or the like. Preferred examples of the suitable liquid cosmetic material applicator include those equipped with an absorbent core-type container, which holds liquid cosmetic material in an absorbent core and has excellent usability, simplicity, and an application property, or a collector-type container, which holds liquid cosmetic material in a lamella, or those in which liquid cosmetic material composition is stored directly in an application liquid storage portion and in which a mechanism of discharging the application liquid is by clicking or by rotation (turning).

In the present disclosure, a preferable liquid cosmetic material applicator includes at least an application portion and an application liquid storage portion, the application portion consisting of a brush whose fibers have non-circular cross sections, and the application liquid storage portion storing the liquid cosmetic material composition of the present disclosure.

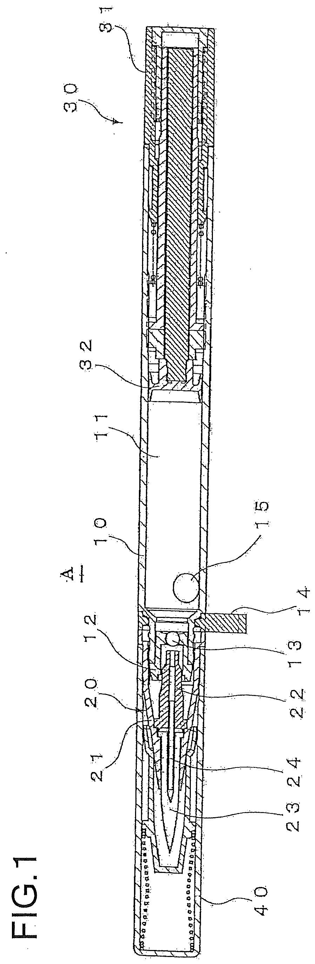

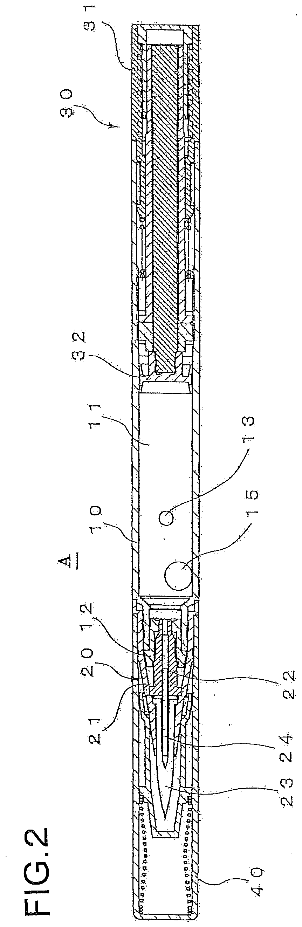

Examples of the liquid cosmetic material applicator of this embodiment include a liquid cosmetic material applicator A illustrated in FIGS. 1 and 2.

As illustrated in FIG. 1, the liquid cosmetic material applicator A of this embodiment includes a storage portion (accommodation portion) 11, a plug 12, and a sealing ball 13. The storage portion (accommodation portion) 11 stores the application liquid which is the liquid cosmetic material composition of the present disclosure having the above configuration, and is provided inside the front of a barrel body 10 which is the applicator main body. The plug 12 is installed in the front end portion of the barrel body 10 and seals the storage portion (accommodation portion) 11 before use. The sealing ball 13 is installed in the plug 12 to maintain the sealed state of the storage portion (accommodation portion) 11, and is removed from the plug 12 to release the storage portion (accommodation portion) 11 from the sealed state, putting the application liquid in a state of being discharged from the storage portion (accommodation portion) 11.

The liquid cosmetic material applicator A includes a stopper 14 detachably installed at the outer circumferential surface of the front end portion of the barrel body 10, and a front barrel 20 that moves in the axial direction by the length of the stopper 14 and joins directly to the barrel body 10 when the stopper 14 is removed at the start of use.

The front barrel 20 is provided with a front barrel body 21, a pipe joint 22, and an application liquid conducting tube 24. The pipe joint 22 is installed at the front barrel body 21 and allows for the supply of the application liquid from the storage portion 11 to a brush (application portion) 23. The application liquid conducting tube 24 connects the pipe joint 22 and the brush (application portion) 23.

With this configuration, in the state illustrated in FIG. 1 (the state before the start of use), the stopper 14 is installed at the barrel body 10, and thus the sealed state of the storage portion (accommodation portion) 11 is maintained by the plug 12 (sealing ball 13) sealing the storage portion (accommodation portion) 11 without the front barrel 20 moving.

As illustrated in FIG. 2, when the stopper 14 is removed at the start of use, the front barrel 20 moves in the axial direction by the length of the stopper 14 and comes into contact with the barrel body 10. That is, the pipe joint 22 of the front barrel 20 and the plug 12 are joined to each other, and the sealing ball 13 installed at the plug 12 is removed by the tip portion of the pipe joint 22.

As a result, the storage portion (accommodation portion) 11 is released from the sealed state, making the application liquid available to be supplied from the storage portion 11 to the brush (application portion) 23.

Moreover, an application liquid discharge mechanism 30 is provided at the rear end portion of the barrel body 10. The application liquid discharge mechanism 30 has a configuration in which a top 31 rotates and moves a piston 32 forward by a predetermined distance, thereby discharging a predetermined amount of the application liquid. Moreover, the brush 23 is covered by a cap 40.

Note that, in FIGS. 1 and 2, the reference numeral 15 denotes a stirring ball for stirring the application liquid. The application liquid discharge mechanism 30 is disclosed in JP 2012-157611 A previously proposed by the present applicants. The application liquid discharge mechanism 30 may be a rotation type in which the application liquid is discharged by rotating the top 31, or may also be a clicking type in which the application liquid is discharged by pressing the top 31.

In the liquid cosmetic material applicator A configured as described above, first, the stopper 14 is removed, and the cap 40 (front barrel body 21) is pushed (moved) toward the rear side of the barrel body 10. As a result, the end of the pipe joint 22 comes into contact with the sealing ball 13, and the sealing ball 13 is removed from a sealing receiver 12, making the application liquid available to be supplied.

Thereafter, the cap 40 is removed, the top 31 is rotated to advance the piston 31 by a predetermined distance, thereby discharging a predetermined amount of the application liquid to the brush 23. Then, the application liquid is applied to an application site on a user.

The shape, structure, material, and the like of the brush 23 to be used are not limited as long as it can be suitably applied to eyeliners, eyeshadows, eyebrow makeup, or the skin. In the present embodiment, the brush is composed of an aggregate of fibers (brush bristles) made of linear synthetic fibers with tapered tips and having non-circular irregular cross sections, and is composed of a plurality of types of brush bristles having different cross-sectional shapes. A brush bristle having an irregular cross section refers to a brush bristle whose plane perpendicular to the axial direction of the brush bristle has a shape other than a circular shape, and examples thereof include those having a cross-sectional shape such as a substantially star shape, a substantially cocoon shape, a substantially cross shape, a substantially triangular shape, a substantially Y shape, a substantially semicircular shape, and a substantially rectangular shape, and those having a shape having at least one or more projections . Examples of the synthetic fibers include synthetic resin fibers obtained by melt-spinning thermoplastic polymers, for example, polyesters such as polyethylene terephthalate, polybutylene terephthalate, and polyethylene terephthalate-isophthalate co-condensation polymer, polyolefins such as polyethylene and polypropylene, or polyamides such as nylon 6, nylon 610, and nylon 612.

In the liquid cosmetic material applicator A configured as described above, when multiple types of bristles with different cross-sectional shapes are used for the brush serving as the application portion, the reduction of voids (gaps) among the bristles is suppressed, giving the brush more strength. Furthermore, the aqueous liquid cosmetic material disclosed above does not skip on the skin even when applied to the skin, and contains a pigment having carbon as a main component and having a low viscosity with little dripping (dropping), and thus can be used to draw intense lines . The multiplier effect of the characteristics of the aqueous liquid cosmetic material and the structural characteristics of the liquid cosmetic material applicator allows the brush to contain sufficient amount of the aqueous liquid cosmetic material that serves as the application liquid, making the application comfortable. Moreover, the composition properties in the present disclosure include at least the flaky pigment A, the flaky pigment B surface-treated with at least one type of crystalline cellulose, the dextrin, the thickening polysaccharide, the emulsion particles containing an acrylate copolymer, and water. As such, even when the composition contains a larger amount of a flaky pigment such as a glittering pigment than the known ones and is prepared into a low-viscosity aqueous blending composition, the result is still an aqueous liquid cosmetic material suitable for makeup cosmetic materials for eyeshadows, eyeliners, eyebrow makeup, mascaras, and the like that can greatly improve the intensity of lines drawn using the cosmetic material without causing aggregation or settling of the flaky pigment while achieving an improved adhering property.

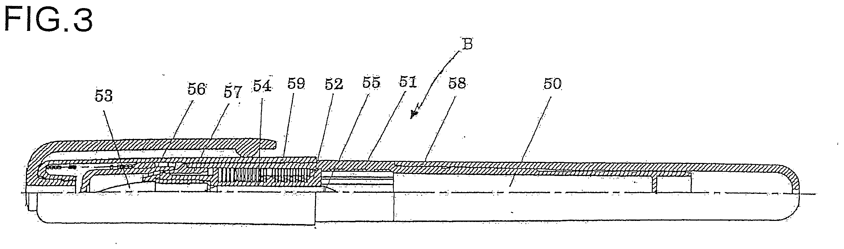

FIG. 3 illustrates a free-ink collector type liquid cosmetic material applicator accommodating the liquid cosmetic material composition of the present disclosure.

In the liquid cosmetic material applicator B, for example, as illustrated in FIG. 3, a liquid cosmetic material composition 50 of the present disclosure is filled in a tank portion 51. The tank portion 51 serves as a barrel body that directly stores the liquid cosmetic material composition 50 without the liquid cosmetic material composition 50 being absorbed in an absorbent core or the like. The front portion of the tank portion 51 contains a lamella 52 (ink reservoir, collector member), which temporarily stores the liquid cosmetic material 50 that is pushed out from the tank portion 51 when the air in the tank portion 51 expands due to a rise in temperature or the like to prevent the tank portion 51 from dripping from the pen tip or air hole. The tip portion of the collector member 52 is provided with a brush-shaped pen tip (brush) 53 that serves as the applicator body. The brush-shaped pen tip (brush) 53 may be the brush 23 having an irregular cross section used in the liquid cosmetic material applicator A above.

Liquid cosmetic material is discharged from the tank portion 51 to the pen tip 53 as follows: the liquid cosmetic material 50 is discharged from the tank portion 51 to the pen tip 53 serving as the application portion via a relay core 55 provided with an application liquid flow path 54 arranged at a center hole of the collector member 52.

Note that, 56 and 57 in FIG. 3 are holder members, 58 is a rear barrel body fixed to the rear portion of the tank portion 51, and 59 is a cap having an inner cap. Alternatively, liquid cosmetic material may be discharged by directly arranging the rear portion of the pen tip 53 within the tank portion 51 without involving the relay core 55.

The liquid cosmetic material applicator B of this embodiment can be made available for use in the same manner as the liquid cosmetic material applicator A by removing the cap 59.

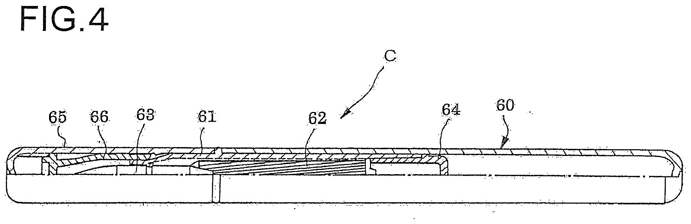

FIG. 4 presents a case where the aqueous liquid cosmetic material of the present disclosure is used in an absorbent core-type liquid cosmetic material applicator.

For example, as illustrated in FIG. 4, the liquid cosmetic material applicator C of this embodiment has the following structure: an inner barrel 61 is provided in a cosmetic applicator body 60, an impregnated body 62 that is an absorbent core impregnated with the liquid cosmetic material composition of the present disclosure is accommodated in the inner barrel 61, an applicator body 63 composed of a brush having an irregular cross section used in the liquid cosmetic material applicator A for applying liquid cosmetic material is provided on the tip side of the impregnated body 62, and a tail plug 64 is fixed to the rear end of the inner barrel 61. Note that 65 denotes a cap body having an inner cap portion 66. The liquid cosmetic material applicator C of this embodiment can be made available for use by removing the cap 65.

The liquid cosmetic material applicator illustrated in FIG. 3 or 4 configured as described above also allows for the brush to contain a sufficient amount of the aqueous liquid cosmetic material of the present disclosure serving as the application liquid, resulting in a liquid cosmetic material applicator offering comfortable application. Note that, in the liquid cosmetic applicators A to C of the above embodiments, a brush with an irregular cross section in which the fibers of the brush have cross sections other than circular is used. However, the brush in each of the above embodiments A to C may be made of linear synthetic fibers that have tapered tips and that may have circular cross sections. This is because, as mentioned above, the aqueous liquid cosmetic material of the present disclosure does not get faint on a skin when applied to the skin, and can be used to draw intense lines with little dripping (dropping) and at a low viscosity.

In the liquid cosmetic material applicator of the above-described embodiments, the example described is a liquid cosmetic material applicator of liquid eyeliner or liquid eyeshadow which is a cosmetic material composition serving as the aqueous liquid cosmetic material of the present disclosure. However, the present disclosure is not limited thereto, and the present disclosure can also be applied to an eyebrow applicator for drawing lines on eyebrows, and drawing lines on the skin. Further, the liquid pressing mechanism of the liquid cosmetic material applicator used in the above embodiments was a rotary feeding type applicator illustrated in FIG. 1, but it may also be a clicking feeding type liquid cosmetic material applicator.

The aqueous liquid cosmetic material of the present disclosure configured as described above is a cosmetic material accommodated in a pen-type applicator having a brush in the application portion, and contains raw materials that are at least the flaky pigment A, the flaky pigment B surface-treated with at least one type of crystalline cellulose, the dextrin, the thickening polysaccharide, the emulsion particles containing an acrylate copolymer, and water combined in suitable contents . This allows for the provision of an aqueous liquid cosmetic material suitable for makeup cosmetic materials for eyeshadows, eyeliners, eyebrow makeup, mascaras, and the like that, even when containing a larger amount of a flaky glittering pigment than the known ones and made into a low-viscosity aqueous blending composition, can still greatly improve the intensity of lines drawn using the cosmetic material without causing aggregation or settling of the flaky pigment while also achieving an improved adhering property.

### Examples

Next, the present disclosure will be described in further detail with reference to examples and comparative examples, but the present disclosure is not limited by the following examples and the like.

### [Examples 1 to 8 and Comparative Examples 1 to 7]

Cosmetic material compositions serving as aqueous liquid cosmetic materials having the blending formulations presented in Table 1 below (blending unit: mass%, total amount: 100 mass%) were prepared by the following method. The viscosity value of each liquid cosmetic material was evaluated using the above measurement method, while the redispersibility over time, the degree of glittering of the lines drawn, and vertical difference over time were evaluated using the following evaluation methods.

These results are shown in Table 1 below.

### (Method for Preparing Aqueous Liquid Cosmetic Material)

A cosmetic composition serving as an aqueous liquid cosmetic material having a blending formulation presented in Table 1 below was prepared according to a commonly used procedure, that is, by adding various flaky pigments serving as coloring materials, other pigments, and a water-soluble acrylic copolymer to purified water serving as a vehicle, dispersing the foregoing using a Labstar (available from Ashizawa Finetech Ltd.) as a disperser, and then adding and mixing the other components.

### Method for measuring viscosity

For each aqueous liquid cosmetic material obtained by the above method, the viscosity at a predetermined shear rate (50 rpm) at a temperature of 25°C using a cone and plate viscometer (ELD viscometer among TV-30 viscometers, standard cone and plate, available from Tokimec) was measured.

### (Method for Evaluating Redispersibility Over Time)

Each liquid cosmetic material was accommodated in a sample bottle and allowed to stand at room temperature (25°C) for one week. Then, after stirring was performed for a certain period of time, the liquid cosmetic material was subjected to sensory evaluation in which how easily the sediment broke apart was visually observed.

Evaluation criteria:
A: The cake breaks apart within 10 seconds.
B: The cake breaks apart in 10 to 30 seconds.
C: The cake breaks apart in 30 to 60 seconds.
D: It takes more than 60 seconds for the cake to break apart.

### (Glittering Feel: Method for Evaluating Degree of Glittering of Lines Drawn)

A pen-type applicator having a brush at an application portion of FIGS. 1 and 2 was used to apply and spread an aqueous liquid cosmetic material on a skin, and the glittering feel (degree of glittering of lines drawn) and the vertical difference (of glittering feel) over time were evaluated by the following test method.

The brush used in Examples of FIGS. 1 and 2 is a tapered pen brush composed of a bundle of polybutylene terephthalate resin fibers each having a diameter of 0.05 to 0.3 mm. Each of the aqueous liquid cosmetic material obtained above was accommodated in the pen-type applicator having a brush at an application portion, applied to the skin, and subjected to a sensory evaluation in which the glittering feel was evaluated in accordance with the following evaluation criteria by visual observation.

Evaluation criteria:
A: The glittering feel is high.
B: The glittering feel is not strong.
C: There is almost no glittering feel.

### [Method for Evaluating Vertical Difference of Glittering Feel]

The applicator was fixed upward and allowed to stand at room temperature (25°C) for one month. Thereafter, application was performed in the same manner as Glittering Feel described, and a sensory evaluation was carried out to visually observe whether or not there was a glittering feel in accordance with the following evaluation criteria.

Evaluation criteria:
A: There is a glittering feel.
B: The glittering feel is weak.
C: There is almost no glittering feel.

**[Table 1]**

| (Total amount: 100 mass%) | | | | | | | | | | | | | | | |
|---|---|---|---|---|---|---|---|---|---|---|---|---|---|---|---|
| | Examples | | | | | | | | Comparative Examples | | | | | | |
| | 1 | 2 | 3 | 4 | 5 | 6 | 7 | 8 | 1 | 2 | 3 | 4 | 5 | 6 | 7 |
| Flaky pigment A-1 *1 | 1.00 | 1.00 | 1.00 | 1.00 | 1.00 | | 1.00 | 1.00 | 4.33 | | 1.00 | 1.00 | 1.00 | 1.00 | 1.00 |
| Flaky pigment (Surface-Treated) B-1 *2 | 3.33 | 3.33 | 3.33 | 3.33 | 3.33 | 3.33 | 3.33 | | | 0.88 | 3.33 | 3.33 | 3.33 | 3.33 | 10.00 |
| Flaky pigment A-2 *3 | | | | | | 1.00 | | | | | | | | | |
| Flaky pigment (Surface-Treated) B-2 *4 | | | | | | | | 3.33 | | | | | | | |
| Red Pigment *5 | | 1.51 | | | | | 1.38 | 1.51 | 1.51 | 1.51 | 1.51 | 1.51 | 1.51 | 1.51 | 1.51 |
| Iron Oxide *6 | 4.48 | | | | | | | | | | | | | | |
| Carbon Black *7 | | | | 1.65 | 1.65 | 1.65 | 0.15 | | | | | | | | |
| Emulsion Particles (Acrylic Copolymer) *8 | 7.76 | 7.76 | 7.76 | 7.76 | 7.76 | 7.76 | 7.76 | 7.76 | 7.76 | 7.76 | 3.00 | 7.76 | 7.76 | 11.00 | 7.76 |
| Dextrins (α-cyclodextrin) | 0.13 | 0.13 | 0.13 | 0.13 | 0.13 | 0.13 | 0.13 | 0.13 | | 0.05 | 0.13 | 0.13 | | | 0.43 |
| Thickening Poly saccharide B (Xanthan Gum) | 0.03 | 0.03 | 0.03 | 0.03 | 0.03 | 0.03 | 0.03 | 0.03 | | 0.01 | 0.03 | | 0.03 | 0.03 | 0.11 |
| Thickener (Crystalline Cellulose) | 0.50 | 0.50 | 0.50 | 0.50 | 0.50 | 0.50 | 0.50 | 0.50 | | 0.21 | 0.50 | 0.60 | 0.60 | 0.60 | 1.67 |
| pH Adjuster *9 | 0.34 | 0.04 | 0.02 | | 0.02 | 0.02 | 0.02 | 0.02 | 0.04 | 0.04 | 0.04 | 0.03 | 0.03 | 0.03 | 0.03 |
| Preservative | 1.91 | 1.91 | 1.90 | 1.90 | 1.90 | 1.90 | 1.91 | 1.90 | 1.91 | 1.91 | 1.90 | 1.91 | 1.91 | 1.91 | 1.91 |
| 1,3-butylene glycol | 10.50 | 10.50 | 10.50 | 10.50 | 10.50 | 10.50 | 10.50 | 10.50 | 10.50 | 10.50 | 10.50 | 10.50 | 10.50 | 10.50 | 10.50 |
| Water (Purified Water) | Balance | Balance | Balance | Balance | Balance | Balance | Balance | Balance | Balance | Balance | Balance | Balance | Balance | Balance | Balance |
| Viscosity (25°C, mPa-s) | 4.0 | 3.8 | 3.4 | 3.7 | 3.7 | 3.7 | 3.9 | 3.5 | 2.7 | 2.5 | 2.5 | 3.5 | 3.0 | 10.0 | 8.0 |
| Redispersibility Over Time | A | A | A | B | A | B | A | A | D | A | B | C | C | B | D |
| Degree of Glittering of Lines Drawn | A | A | A | A | A | A | A | A | A | C | A | A | A | C | A |
| Vertical Difference Over Time | A | A | A | A | A | A | A | A | C | C | C | B | B | A | B |

*1 to *9 in Table 1 above are as described below.
*1: Cosmicolor Crystal SL (available from Toyo Aluminum K.K.), average particle size 64 µm
*2: (Production Example) Cosmicolor Frost SL (available from Toyo Aluminum K.K.) surface-treated with crystalline cellulose [(Ceolus RC-591 (available from Asahi Kasei Corporation)] by spray drying, average particle size of smooth surface 10 to 70 µm, surface coating ratio 2%
*3: Cosmicolor Frost SL (available from Toyo Aluminum K.K.), average particle size 20 µm
*4: VISIONAIRE SPARKING SILVER SEA (available from ECKART) surface-treated with crystalline cellulose [(Ceolus RC-591 (available from Asahi Kasei Corporation)] by spray drying, average particle size of smooth surface 20 to 150 µm, surface coating ratio 2%
*5: DK D&C RED, available from DAITO KASEI KOGYO CO., LTD.
*6: Red iron oxide No. 216P, available from DAITO KASEI KOGYO CO., LTD.
*7: DK Black No. 2, available from DAITO KASEI KOGYO CO., LTD.
*8: Acrylate copolymer: Yodosol GH800F, solids content 45%, available from Akzo Nobel (the amounts presented in the table are solids content)
*9: AMP citric acid

The results in Table 1 above indicate that the aqueous liquid cosmetic materials of Examples 1 to 8, which fall within the range of the present disclosure, have excellent over time redispersibility, glittering feel, as well as vertical difference (glittering feel) over time, compared to Comparative Examples 1 to 7, which fall outside the range of the present disclosure.

### Industrial Applicability

Provided is an aqueous liquid cosmetic material suitable for makeup cosmetic materials for eyeshadows, eyeliners, eyebrow makeup, mascaras, and the like that. Even when containing a larger amount of a flaky pigment such as a glittering pigment than the known ones and made into a low-viscosity aqueous blending composition, the aqueous liquid cosmetic material does not cause the flaky pigments to be aggregated or settled, stillimproves the intensity of lines drawn, and improves adhering property.

### Reference Signs List

- 1: Applicator
- 10: Barrel Body
- 11: Storage Portion (Accommodation Portion)
- 12: Plug
- 13: Sealing Ball
- 14: Stopper
- 20: Front Barrel
- 21: Front Barrel Body
- 23: Brush (Application Portion)

## Claims

1. An aqueous liquid cosmetic material accommodated in a pen-type applicator having a brush at an application portion,
the aqueous liquid cosmetic material comprising at least a flaky pigment A, 1 to 9 mass% of a flaky pigment B that is surface-treated with at least one of crystalline celluloses, a dextrin, a thickening polysaccharide, emulsion particles containing 4 to 10 mass% of an acrylate copolymer in terms of solid content, and water.

2. The aqueous liquid cosmetic material according to claim 1, wherein the dextrin is at least one selected from Group X below:
Group X: powdered syrup, maltodextrin, dextrin, and cyclic dextrin.

3. The aqueous liquid cosmetic material according to claim 1, wherein the thickening polysaccharide is at least one selected from Group Y below:
Group Y: xanthan gum, carrageenan, guar gum, locust bean gum, tamarind gum, cationic guar gum, and quince seed.

4. The aqueous liquid cosmetic material according to any one of claims 1 to 3, further comprising a pH adjuster.

5. The aqueous liquid cosmetic material according to any one of claims 1 to 4, wherein the flaky pigment A is a silica-coated pigment.

6. The aqueous liquid cosmetic material according to any one of claims 1 to 5, wherein the flaky pigment A comprises an untreated flaky pigment having a different particle size.

7. The aqueous liquid cosmetic material according to any one of claims 1 to 6, wherein the flaky pigment B comprises a flaky pigment having a different particle size and surface-treated with crystalline cellulose.

8. The aqueous liquid cosmetic material according to any one of claims 1 to 7, wherein a particle size of the flaky pigment A and a particle size of the flaky pigment B are each 1 to 50 µm or 51 to 100 µm, and a difference therebetween is 30 µm or greater.
